# EUROPEAN PATENT SPECIFICATION

(11) **EP 2 276 356 B1**
(45) Date of publication and mention of the grant of the patent: **19.06.2013**
(21) Application number: 09732502.1
(22) Date of filing: 06.04.2009
(51) Int. Cl.: A23L 1/22, A61Q 11/00, A61Q 19/00

(54) **WARMING SENSATE COMPOSITION**
WÄRMENDE WAHRNEHMUNGSSTOFFZUSAMMENSETZUNG
COMPOSITION A SENSATION DE RECHAUFFEMENT

(30) Priority: 15.04.2008 US 45138
(43) Date of publication of application: 26.01.2011
(73) Proprietor: Firmenich S.A., 1211 Geneva 8 (CH)
(72) Inventor: DESIERTO, Bernadita, Belle Mead, New Jersey 08502 (US); LE, Anh, Princeton, New Jersey 08540, (US); STANIEC, Nicole, Ewing, New Jersey 08628 (US); ZANONE, John, Towaco, New Jersey 07082 (US)
(74) Representative: Dale, Gavin Christopher
(86) International application number: PCT/IB2009/051435
(87) International publication number: WO 2009/127992

(56) References cited:
- EP-A- 1 568 365
- EP-A- 1 632 226
- WO-A-98/47482
- WO-A-2004/043169
- WO-A-2004/045590
- WO-A-2005/048743
- WO-A-2005/117981
- WO-A-2006/109241
- GB-A- 2 068 730
- US-A- 5 711 937
- US-A1- 2003 215 532
- DATABASE WPI Week 198523 Thomson Scientific, London, GB; AN 1985-138982 XP002537221 & JP 60 075427 A (LION CORP) 27 April 1985 (1985-04-27)
- 'Peppermint (Mentha piperita L.)', [Online] Retrieved from the Internet: <URL:http://www.uni-graz.at/~katzer/engl/Me nt_pip.html>

## Description

### Technical Field

The present invention relates to the field of flavor and taste. In particular, the invention relates to compositions and to edible products comprising the compositions which impart a warming sensation when consumed.

### Background of the Invention

The particular sensations mediated by the trigeminal nerve, such as cooling, warming, pungent and salivating are becoming more appreciated by consumers in food, body care and perfumed products. These trigeminal compounds are capable of providing unique characteristics to a large variety of foods and they are even perceived on the skin of the body. Accordingly, US6780443, US6890567 and US6899901 (all to Takasago International Corporation) disclose compositions comprising a cooling sensate, a warming sensate and a tingling sensate, the purpose of which is to provide an initial strong sensation in the oral cavity, such as tingling or stinging.

It is also known to combine compounds known to possess flavor and/or sensate compounds to produce new active ingredients having altered properties. For example, WO 98/47482 discloses formulations for cough drops which include a physiological cooling agent. WO 2006/109241 reports a hot/cooling ingredient weight ratio of 1:70.3 and reports a hot sensation, but is completely silent as regards the problem of providing a chest warming effect

None of these documents addresses the issue of providing a warming sensation in the mouth, throat and chest.

In the pharmaceutical industry, over the counter ("OTC") type medicines, particularly analgesics such as cold relief products, and especially cough syrups, often contain ethanol, which not only acts as a solvent for the active ingredients but, more importantly for the consumer, delivers a mouth, throat and chest-warming sensation, which serves to reinforce the soothing effect delivered by the medicine. However, the use of ethanol in such products may not always be desirable. For instance, some consumers prefer to avoid its consumption for health, religious or other reasons and some consumers are known to suffer an allergic reaction to it and are consequently obliged to avoid its consumption. Therefore, it would be a clearly desirable consumer benefit to provide a product that achieves substantially the same warming sensation as provided by ethanol and so can replace, at least partly, the ethanol content therein.

US6780443, US6890567 and US6899901 do not address this issue and even disclose numerous compositions containing very high levels of ethanol

In another aspect, the composition should preferably avoid delivering spicy- or off-notes since these will be undesirable in analgesic consumer products such as cough mixtures, where such spiciness may even serve to further irritate a sore throat.

Therefore, it is a preferred objective of the present invention to provide a warming effect devoid of strong spicy notes. In particular, it is an objective to provide a flavour sensation composition providing a clean, warming trigeminal effect capable of replacing substantially or entirely ethanol in a product, especially, an analgesic product.

In particular, it is an objective of the present invention to provide a composition in which the ingredients synergistically interact with eachother to provide a warming sensation in a way that the sensation is perceived in not only the mouth but also the throat and the chest.

### Summary of the Invention

Remarkably, the present inventors have found that a composition comprising a hot component and a cooling component in a specific ratio is capable of providing a clean warming sensation that is perceived not only in the mouth but also in the throat and chest, and so is effective as a replacement for ethanol where it is used to this effect.

Accordingly, the present invention provides, in a first aspect, a warming sensate composition comprising
(i) a hot component,
(ii) a cooling component,
(iii) optionally a bitter component, and
(iv) a solvent for components (i) to (iii)
wherein the weight ratio of (i) to (ii) is from 1:3 to 1.35 and, if the cooling component comprises menthol or a mentholic flavor compound, said menthol or mentholic flavor compound is present at a level of 2% by weight or less of the warming sensate composition, with the proviso that the bitter component (iii) is present at less than 0.15% by weight based on the total weight of the composition, and the solvent (iv) comprises less than 10wt% ethanol and from 20 to 99.9% of propylene glycol, based on the total weight of the warming sensate composition.

In another aspect, the present invention provides the use of a composition as defined above to deliver a warming sensation to the mouth, throat and chest of a consumer.

In a further aspect, the invention provides a food product, an oral care product, a body care product or a pharmaceutical product comprising the warming sensate composition as defined above.

### Detailed Description of the Invention

The composition of the present invention comprises a hot component and a cooling component wherein the weight ratio of hot component to cooling component is from 1:3 to 1:35.

The composition is preferably used in orally ingestible products. More preferably, it is used in pharmaceutical products, even more preferably analgesic preparations. The product may be provided in the form of a solid, liquid, gel, suspension or the like. Particularly preferred are syrups and liquid-centred throat lozenges, where ethanol is conventionally used to deliver a warming sensation to the mouth, throat and chest.

Nevertheless, it is envisaged that the composition of the invention may also be used in other consumer products where the warming sensation referred to above is desired. Therefore, the composition can also be used in beverages, which may be either alcoholic or non-alcoholic, chewing gums, confectionary, savoury foods and so on.

For instance, certain beverages are consumed warm or hot and so it may be desirable to achieve such a warming effect even in the absence of a means for heating the beverage. An example is mulled wine, an alcoholic beverage consumed warm to give a pleasant warming effect. By incorporating the composition of the invention, the level of alcohol can even be reduced whilst maintaining the desired warming effect.

### Hot Sensate Component

The composition according to the invention comprises a hot component consisting of one or more hot sensate compounds. By hot sensate compound, it is meant a compound which provides a hot, warming sensation when brought into contact with the oral cavity or the skin. In particular, the hot component provides a hot, warming sensation mediated by the trigeminal nerve. The hot compound is believed to stimulate primarily receptor TRPV1.

The hot compound is preferably a purified compound, but may also be provided in the form of an extract.

Examples of suitable hot sensate compounds include those having a structure according to the following formula: 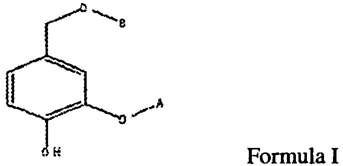 or an acceptable salt thereof wherein A is an unsubstituted, branched or straight-chained C₁-C₃ alkyl group and B is a hydrogen, or an unsubstituted, branched or straight-chained C₁-C₇ alkyl group. In one embodiment A represents a methyl group. In a preferred embodiment, A represents a methyl group and B represents a C₂-C₄ alkyl group. In a particularly preferred embodiment, the warming sensate is selected from vanillyl butyl ether and vanillyl ethyl ether. Vanillyl butyl ether is commercially available under the trade name Hotact^{®} VBE (ex Takasago, Inc). Other hot sensate compounds suitable for use in the composition of the present invention include gingerol, vanillyl propyl ether, vanillyl pentyl ether, vanillyl hexyl ether, vanillyl butyl ether acetate, 4-(1-menthoxymethyl)-2-phenyl-1,3-dioxolan, 4-(1-menthoxymethyl)-2-(3',4'-dihydroxyphenyl)-1,3-dioxolan, 4-(1-menthoxymethyl)-2-(2'-hydroxy-3'-methoxyphenyl)-1,3-dioxolan, 4-(1-menthoxymethyl)-2-(4'-methoxyphenyl)-1,3-dioxolan, 4-(1-menthoxymethyl)-2-(3',4'-methylenedioxyphenyl)-1,3-dioxolan, 4-(1-menthoxymethyl)-2-(3'- methoxy-4'-hydroxyphenyl)-1,3-dioxolan, red pepper oil, red pepper oleoresin, ginger oleoresin, nonylic acid vanillyl amide, jambu oleoresin, Zanthoxylum piperitum extract, sanshool I, sanshool II, sanshoamide, black pepper extract, chavicine, piperine and spilanthol. Other suitable hot sensate compounds are disclosed in, for example, US 6,780,443. The hot component preferably excludes capsaicin since the spiciness perceived by the consumer of such an ingredient is not desirable in the present instance. If capsaicin is to be included, then it is preferably present at a level of less than 0.5% by weight based on the weight of the warming sensate composition.

### Cooling Sensate Component

The composition according to the invention further comprises a cooling component consisting of one or more cooling sensate compounds, with the proviso that, in the finished product, there is no noticeable mentholic taste. By cooling sensate compound, it is meant any compound which, when brought in contact with the skin or oral cavity, provides a cooling sensation, which is mediated by the trigeminal nerve. The cooling compound primarily activates the TRPM8 receptor.

The cooling component is preferably a purified compound, but may also be provided in the form of an extract.

Examples of suitable cooling sensate compounds include (3S,5R,6S,9R)-6-isopropyl-3,9-dimethyl-1,4-dioxaspiro[4.5]decan-2-one and (3S,5S,6S,9R)-6-isopropyl-3,9-dimethyl-1,4-dioxaspiro[4.5]decan-2-one (both marketed under the name cubebol, Firmenich SA), compounds having a structure according to the following formula: 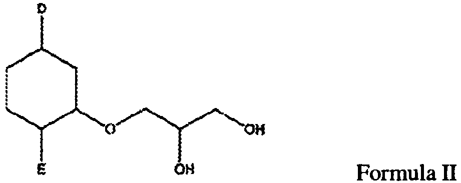 or salts thereof, wherein D is a straight chained or branched, unsubstituted C₁-C₄ alkyl or alkenyl group and E is a straight chained or branched, hydroxy-substituted or unsubstituted C₁-C₄ alkyl group, such as (3-1-menthoxypropane-1,2-diol), available commercially under the name Coolact^{®} 10 (ex Takasago, Inc.), menthol, menthone, camphor, pulegol, isopulegol, cineol, mint oil, peppermint oil or fractions thereof, spearmint oil, eucalyptus oil, N- alkyl-p-menthane-3-carboxamide, 3-1-menthoxy-2-methylpropane-1,2-diol, p-menthane-3,8-diol, 2-1-menthoxyethane-1-ol, 3-1-menthoxypropane-1-ol, 4-1-menthoxybutane-1-ol, 1-(2-hydroxy-4-ethylcyclohexyl)-ethanone, menthyl 3-hydroxybutanoate, menthyl lactate, menthone glycerin ketal, 2-(2-1-menthyloxyethyl)ethanol, menthyl glyoxylate, N-methyl-2,2-isopropylmethyl-3-methylbutanamide, menthyl 2-pyrrolidone-5-carboxylate, monomenthyl succinate, alkali metal salts of monomenthyl succinate, and alkali earth metal salts of monomenthyl succinate, monomenthyl glutarate, alkali metal salts of monomenthyl glutarate, alkali earth metal salts of monomenthyl glutarate, N-[[5-methyl-2-(1-methylethyl) cyclohexyl]carbonyl] glycine, p-menthane-3-carboxylic acid glycerol ester, Menthol propylene glycol carbonate; Menthol ethylene glycol carbonate, and 2-(1-methylpropyl)-1-cyclohexanone (Freskomenthe®, Givaudan). Other cooling sensates are disclosed in US Patent Nos. 7,030,273 and 6,780,443.

If it is desired to use, as the cooling sensate, a compound which delivers a mentholic flavor, it is essential that this is present in the finished product at below the threshold at which a consumer is capable of detecting the menthol flavor. Menthol or the mentholic flavor compound is present at a level of 2% by weight or less of the warming sensate composition. This is because the perception by a consumer of the throat and chest warming effect, which is achievable by the combination of cooling, heating and optionally tingling components, is significantly diminished when the consumer also perceives a menthol flavor. Thus, in one embodiment, the composition may be substantially, more preferably entirely free of menthol and menthol derivatives.

### Bitter Component

Whilst not essential, it has been found that the warming sensation in the throat and chest can be accentuated by the presence of low levels of a bitter component. Accordingly, it is preferred that the composition of the invention comprises a bitter component. The bitter component is a compound or, more generally, an extract having bitter organoleptic properties. Bitterness is one of five recognized basic tastes (besides sweet, salty, sour and umami) and is mediated by taste buds at the back of the tongue. It can readily be attributed to compounds, ingredients, for example. In a preferred embodiment of the invention, the bitter component is selected from bitter triterpenes, glucosides of monoterpenes, sesquiterpenic lactones, humulone, lupulone, flavonones, quinines, and mixtures of these. Examples for bitter plant extracts include Quassia extract (also called bitter ash extract, FEMA No. 2971), quinquina extract, chamomile oils (FEMA No. 2272 to 2274), gentian root extract (FEMA No. 2506), hops extracts and oils (FEMA No. 2578 to 2580, artichoke leaves oil and so on.

Surprisingly, the accentuating effect can be achieved with a very low level of the bitter component. For instance, it is present at levels below 0.15% by weight of the warming sensate composition, may be even below 0.1%, or even below 0.08%. The use of very low levels is advantageous since the risk of a consumer perceiving bitterness is dramatically reduced.

### Solvent

The composition of the present invention comprises at least one solvent, or a mixture of solvents, to dissolve the different components of the composition according to the invention with the proviso that the solvent comprises less than 10wt% ethanol based on the total weight of the sensate composition. Preferably, the solvent comprises less than 5wt%, more preferably less than 2wt%, most preferably less than 1wt% ethanol, or is even substantially free of ethanol. Solvents of current use for the preparation of the sensate composition include, for instance, benzyl alcohol, propylene glycol, triacetine, neobee, vegetable oils or limonene. Propylene glycol is most preferred. A mixture of solvents may be useful to dissolve hydrophobic and hydrophilic components in the same solution. In this case, ethanol may be present but at the very low levels stipulated above. For example, the components of the present invention may be in a solution comprising 20-99.9%, preferably 40-99.9%, more preferably 80-99.9%, even more preferably 90-99.9% of solvent. In the context of the present invention, percentages are percentages by weight, unless otherwise indicated. Similarly, if proportions are indicated as parts, parts by weight are meant.

The composition of the invention may further comprise other ingredients typically used in flavor compositions. For example, the composition may comprise further flavours. The composition may also comprise adjuvants that allow the composition to meet technical requirements, such as stability or tonality persistence. Today, the range of products types and product formulations that are flavoured has become so extensive and subjected to frequent changes that an approach made on a product-by-product basis and on the definition for each case of the adjuvants that can be used is impractical. This is why a list of adjuvants currently used in flavouring formulations is not given here. However, a skilled person in the art, for example a flavourist is capable of choosing these ingredients as a function of the product to be flavoured and of the nature of the flavouring ingredients contained in the formulation.

The sensate composition comprises a hot component comprising one or more hot ingredients and a cooling component comprising one or more cooling ingredients wherein the weight ratio of hot to cooling components is from 1:3 to 1:35, and most preferably from 1:7 to 1:35.

Below 1:3, it has been found that the chest warming effect is not achieved whereas above 1:100, the amount of cooling component negates the warming effect and so it cannot be clearly perceived. Furthermore, above a ratio of 1:100, the presence of even small amounts of "mentholic" compounds may give rise to a noticeable mentholic flavor.

Furthermore, it is preferred that the total amount of cooling and hot components is at least 0.3wt%, based on the total weight of the product in which it is present. Below, this level it is found that the chest warming effect is barely noticeable. By contrast, it is preferred that the total amount of cooling and hot components does not exceed 1wt% based on the total weight of the product in which it is present since this is found to have an undesirable stinging sensation.

The composition of the invention can be made by simply mixing all components of the invention with a solvent at room temperature, at the weight-ratios mentioned above.

In one aspect, the present invention provides of a composition according to the invention to provide a warming flavor to a consumable product, more preferably to providing a throat and chest-warming flavor.

In another aspect, the present invention provides a method for imparting chest and throat warming sensation to a consumable product, the method comprising the steps of adding to the consumable product the composition of the present invention.

In the above uses and method, the composition of the invention may be used directly as it is or added to food products as described below.

The composition of the invention may directly be added to a consumable product at the stage where flavours are generally added. If convenient, a liquid composition may be encapsulated in a matrix, such as a carbohydrate, a phospholipid, or a protein matrix, such as a gelatine matrix, for example, or any bioactive matrix, in order to provide a shelf-stable, dry form of the flavor composition of the invention.

Therefore, in an embodiment, the present invention provides a delivery system comprising the composition of the present invention. Preferably, the delivery system is in the form of a particulate composition and/or a powder. For example, the composition may be encapsulated following standard procedures in spray-dried powders. For example, the composition may be encapsulated in a matrix, which is in a glassy state at room temperature. For example, the composition of the invention may be encapsulated in rod-like granules, the preparation of which is disclosed in US 4,707,367.

The composition of the invention may be used in liquid products and/or in products that are solid. In a preferred embodiment, the product is a liquid since the chest and throat warming effect is found to be more readily achievable in such a product. The consumable product is preferably selected from the group consisting of a liquid pharmaceutical product, such as a liquid analgesic, a beverage, an alcoholic drink, a dairy product, a soy product, a soup, a dressing, a sauce, a dip, and the like.

In one aspect, the present invention provides an oral care product according to the invention. For example, the product may be a toothpaste, a tooth-gel, a mouthwash.

In another aspect, the present invention provides a pharmaceutical product comprising the flavor composition according to the invention. Examples of such products are syrups, lozenges, chewing gums, and drugs provided in the form of tablets or capsules.

The examples below further illustrate the invention without limiting its scope. Samples according to the invention are denoted by a number and comparative samples are denoted by a letter. All amounts are denoted in % by weight unless otherwise stated.

### Example 1

### Preparation of a Sensate Composition

In preparing the sensate compositions, the ingredients described in table 1 were used.

**Table 1**

| Ingredient | Characteristics | Origin |
|---|---|---|
| Anethol | Cooling | Polarome, Inc |
| Menthol Nat. | Cooling | Aldrich |
| Mint Piperita | Cooling | Idaho, USA |
| Cooling Agent 10 | Cooling | Takasago, Inc |
| Coolact P | Cooling | Takasago, Inc |
| Hotact VBE | Hot | Takasago, Inc |
| WS3 | Cooling | Lyondell Chemical Company |
| WS23 | Cooling | Lyondell Chemical Company |
| Piperine | Hot | Aldrich |

| | | |
|---|---|---|
| Sample A was prepared by mixing together 5 parts Cooling agent 10, 5 parts Coolact P and 25 parts Hotact VBE in 965 parts propylene glycol solvent. Sample B was prepared by mixing together 100 parts menthol, 125 parts piperine, 125 parts WS3, 375 parts Hotact VBE in 9275 parts propylene glycol solvent. Sample C was prepared by mixing together 55 parts anethol polarome, 167 parts menthol and 333 parts mint piperita in 445 parts triacetine. Sample D was prepared by mixing 100 parts WS23 in 900 parts propylene glycol solvent. Compositions E, F and 1 to 4 were prepared by mixing various quantities of samples A, B, C and D together with a defined amount of an aqueous 5% sucrose solution. The amounts are given in the following table. | | |

**Table 2**

| Composition | Sucrose soln. | A | B | C | D | Other | Wt Ratio Hot:Cool |
|---|---|---|---|---|---|---|---|
| E | 99.80 | 0.05 | 0.05 | - | - | 0.10* | 2.14:1 |
| F | 99.72 | 0.08 | 0.08 | - | 0.12 | - | 1:2.43 |
| 1 | 99.70 | 0.05 | 0.05 | 0.20 | - | - | 1:30 |
| 2 | 99.63 | 0.08 | 0.05 | 0.08 | - | 0.16* | 1:9.8 |
| 3 | 99.64 | 0.08 | 0.08 | - | 0.20 | - | 1:3.8 |
| 4 | 99.36 | 0.12 | 0.08 | 0.20 | 0.24 | - | 1:19.6 |

| | | | | | | | |
|---|---|---|---|---|---|---|---|
| * Cherry Flavour (ex Firmenich, code 057679A) | | | | | | | |

The samples were then evaluated by a team of 6 trained panelists. Each panelist was asked to describe the effect on the tongue, in the mouth, in the throat and in the chest. The results are given in the following table:

**Table 3**

| Sample | Evaluation |
|---|---|
| E | Cherry, sweet |
| | Hot in the mouth and biting (tingling) on the tongue |
| | No chest warming |
| F | No chest warming |
| | Weak |
| 1 | Gradually perceived chest warming |
| 2 | Cherry, sweet and cooling |
| | Hot in the mouth and biting (tingling) on the tongue |
| | Chest warming |
| 3 | Good chest warming |
| 4 | Chest warming |

The results demonstrate that the desired warming effect in the chest is achieved when the weight ratio of hot to cool ingredients is 1:3 or greater. The results also demonstrate that the effect is not due to the presence of the propylene glycol solvent.

### Example 2

### Candy Comprising a Chest-Warming Sensate Composition

Various samples of syrups are prepared with different warming compositions according to the procedure below.

Isomalt (100g) and water (30g) are mixed and heated to 160-165°C in a copper pan. At 165°C, the copper pan is removed from the gas frame and placed in a warm water bath (40°C). After a few seconds, the copper pan is removed and the temperature checked. When the temperature reaches 135°C, the mixture comprising is further complemented with 1% of an aqueous solution containing Aspartame (10%) and Acesulfame-K (5%). Thereafter, sample 1 is added to provide a composition comprising a total amount of hot and cooling ingredients of 0.3wt% based on the total weight of syrup. The cooked mass is then poured at ambient temperature (at less than 40% relative humidity) into appropriate Teflon® molds and allowed to cool to provide candies comprising the chest warming composition.

### Example 3

### Sugar-free chewing gum comprising the warming composition of the invention

Sugar-free chewing gums are prepared by the following standard procedure: Crystalline sorbitol, Acesulfame-K and aspartame are blended in a Turbula blender. Half the blend is mixed with a pre-warmed Sierra gum base (Cafosa) in a Winkworth sigma-blade mixer at 50-55°C for 2 minutes. The remaining powder blend is then added along with a humectant syrup (Lycasin^{®} 80/55, Sorbit^{®}, glycerin) and mixed for a further 7 minutes. Finally, the sample 2 is added and mixed for one minute at a dosage of 0.35% based on the total weight of chewing gum.

### Example 4

### Savory applications using an invention's composition

A dry blended mixture flavor was prepared according to the following composition:

| **Component** | |
|---|---|
| Salt | 20.00% |
| MSG | 5.00% |
| Ribonucleotide | 0.20% |
| Sugar | 10.00% |
| Yeast Extract Powder | 10.00% |
| Chili Powder | 3.00% |
| Onion Powder | 2.00% |
| Garlic Powder | 2.00% |
| Tomato Powder | 5.00% |
| Whey Powder | 42.30% |
| Paprika Oleoresin | 0.08% |
| Neobee | 0.22% |
| Sample 3 | 0.20% |
| | 100.00% |

## Claims

1. A warming sensate composition comprising
(i) a hot component,
(ii) a cooling component,
(iii) optionally a bitter component, and
(iv) a solvent for components (i) to (iii)
wherein the weight ratio of (i) to (ii) is from 1:3 to 1: 35 and, if the cooling component comprises menthol or a mentholic flavor compound, said menthol or mentholic flavor compound is present at a level of 2% by weight or less of the warming sensate composition, with the proviso that the bitter component (iii) is present at less than 0.15% by weight based on the total weight of the composition, and the solvent (iv) comprises less than 10wt% ethanol and from 20 to 99.9% of propylene glycol, based on the total weight of the warming sensate composition.

2. A composition according to claim 1, wherein the weight ratio of (i) to (ii) is from 1:7 to 1:35.

3. A composition according to claim 1 or claim 2, wherein the total amount of (i) and (ii) is at least 0.3wt%, based on the total weight of a consumable product in which it is present.

4. A composition according to claim 1 or claim 2, wherein the total amount of (i) and (ii) is no greater than 1wt%, based on the total weight of a consumable product in which it is present.

5. A composition according to any one of the preceding claims, in which the hot component agent is selected from the group consisting of piperine, pelargonyl vanillyl amide, vanillyl butyl amide, vanillin butyl ether, eugenol, gingerol, polygodial, shogoal, galangal acetate, capsaicin (N-(4-hydroxy-3-methoxybenzyl)-8-methylnonanamide and/or (6E)-N-(4-hydroxy-3-methoxybenzyl)-8-methyl-6-nonenamide), capsaicin analogs and mixtures comprising two or more of these.

6. A composition according to any one of the preceding claims, in which the cooling component is selected from the group consisting of menthol, menthyl succinate, menthyl lactate, p-menthane-3,8-diol, 8-p-menthen-3-ol, 3-(3'-P-menthanyloxy)-1,2-propanediol, menthone glycerol ketal, 2-(1-methylpropyl)-1-cyclohexanone, N-ethyl-3-P-menthanecarboxamide, aspartic acid, N-(4-hydroxy-3-methoxybenzyl)nonanamide, 5-[5-(1,3-benzodioxol-5-yl)-1-(1-piperidinyl)-2,4-pentadien-1-one, 6-isopropyl-3,9-dimethyl-1,4-dioxaspiro[4.5]decan-2-one, 2-isopropyl-N,2,3-trimethylbutanamide, and, 7-isopropyl-4,10-dimethyl-tricyclo[4.4.0.0(1,5)]decan-4-ol, 3-menthyl-3,6-dioxaheptanoate, 3-menthyl methoxyacetate, 3-menthyl-3,6,9-trioxadecanoate, 3-menthyl-(2-hydroxyethoxy)acetate, menthyl-11-hydroxy-3,6,9-trioxadecanoate, 3S,SR,6S,9R)-6-isopropyl-3,9-dimethyl-1,4-dioxaspiro[4.5]decan-2-one, (3S,5S,6S,9R)-6-isopropyl-3,9-dimethyl-1,4-dioxaspiro[4.5]decan-2-one, 2,3-dihydroxypropyl (1R,2S,SR)-2-isopropyl-5-methylcyclohexane carboxylate, (3S,5R,6S,9R)-6-isopropyl-3,9-dimethyl-1,4-dioxaspiro[4.5]decan-2-one, (3S,SS,6S,9R)-6-isopropyl-3,9-dimethyl-1,4-dioxaspiro[4.5]decan-2-one, (1R,2S,SR)-N-(tert-butyl)-2-isopropyl-5-methylcyclohexanecarboxamide and mixtures comprising two or more of these.

7. Use of a composition as defined in any one of the previous claims to deliver a warming sensation to the mouth, throat and chest of a consumer.

8. A food product, an oral care product, a body care product or a pharmaceutical product comprising the warming sensate composition according to any of claims 1 to 6.

9. A product according to claim 8, in the form of a liquid pharmaceutical product, a beverage, an alcoholic drink, a dairy product, a soy product, a soup, a dressing, a sauce or a dip.

10. A product according to claim 9 in the form of a liquid analgesic preparation.

## Patentansprüche

1. Wärmend wahrgenommene Zusammensetzung, umfassend
(i) eine scharfe Komponente,
(ii) eine kühlende Komponente,
(iii) wahlweise eine bittere Komponente und
(iv) ein Lösungsmittel für die Komponenten (i) bis (iii),
wobei das Gewichtsverhältnis von (i) zu (ii) von 1:3 bis 1:35 ist und, wenn die kühlende Komponente Menthol oder eine Verbindung mit mentholischem Aroma umfasst, das Menthol oder die Verbindung mit mentholischem Aroma mit einem Niveau von 2 Gew.-% oder weniger von der wärmend wahrgenommenen Zusammensetzung vorhanden ist, mit der Maßgabe, dass die bittere Komponente (iii) mit weniger als 0,15 Gew.-%, bezogen auf das Gesamtgewicht der Zusammensetzung, vorhanden ist und das Lösungsmittel (iv) weniger als 10 Gew.-% Ethanol und von 20 bis 99,9% Propylenglycol, bezogen auf das Gesamtgewicht der wärmend wahrgenommenen Zusammensetzung, umfasst.

2. Zusammensetzung gemäß Anspruch 1, wobei das Gewichtsverhältnis von (i) zu (ii) von 1:7 bis 1:35 ist.

3. Zusammensetzung gemäß Anspruch 1 oder Anspruch 2, wobei die Gesamtmenge von (i) und (ii) mindestens 0,3 Gew.-%, bezogen auf das Gesamtgewicht eines konsumierbaren Produkts, in welchem sie vorhanden sind, beträgt.

4. Zusammensetzung gemäß Anspruch 1 oder Anspruch 2, wobei die Gesamtmenge von (i) und (ii) nicht größer als 1 Gew.-%, bezogen auf das Gesamtgewicht eines konsumierbaren Produkts, in welchem sie vorhanden sind, ist.

5. Zusammensetzung gemäß einem der vorhergehenden Ansprüche, in welcher das Mittel der scharfen Komponente aus der Gruppe, bestehend aus Piperin, Pelargonylvanillylamid, Vanillylbutylamid, Vanillinbutylether, Eugenol, Gingerol, Polygodial, Shogaol, Galangalacetat, Capsaicin (N-(4-Hydroxy-3-methoxybenzyl)-8-methylnonanamid und/oder (6E)-N-(4-Hydroxy-3-methoxybenzyl)-8-methyl-6-nonenamid), Capsaicin-Analogen und Gemischen, umfassend zwei oder mehrere von diesen, ausgewählt ist.

6. Zusammensetzung gemäß einem der vorhergehenden Ansprüche, in welcher die kühlende Komponente aus der Gruppe, bestehend aus Menthol, Menthylsuccinat, Menthyllactat, p-Menthan-3,8-diol, 8-p-Menthen-3-ol, 3-(3'-P-Menthanyloxy)-1,2-propandiol, Menthonglycerolketal, 2-(1-Methylpropyl)-1-cyclohexanon, N-Ethyl-3-P-menthancarboxamid, Asparaginsäure, N-(4-Hydroxy-3-methoxybenzyl)nonanamid, 5-[5-(1,3-Benzodioxol-5-yl)-1-(1-piperidinyl)-2,4-pentadien-1-on, 6-Isopropyl-3,9-dimethyl-1,4-dioxaspiro[4.5]decan-2-on, 2-Isopropyl-N,2,3-trimethylbutanamid und 7-Isopropyl-4,10-dimethyl-tricyclo[4.4.0.0(1,5)]decan-4-ol, 3-Menthyl-3,6-dioxaheptanoat, 3-Menthylmethoxyacetat, 3-Menthyl-3,6,9-trioxadecanoat, 3-Menthyl-(2-hydroxyethoxy)acetat, Menthyl-11-hydroxy-3,6,9-trioxadecanoat, 3S,SR,6S,9R)-6-Isopropyl-3,9-dimethyl-1,4-dioxaspiro[4.5]decan-2-on, (3S,5S,6S,9R)-6-Isopropyl-3,9-dimethyl-1,4-dioxaspiro[4.5]decan-2-on, 2,3-Dihydroxypropyl(1R,2S,5R)-2-isopropyl-5-methylcyclohexancarboxylat, (3S,5R,6S,9R)-6-Isopropyl-3,9-dimethyl-1,4-dioxaspiro[4.5]decan-2-on, (3S,SS,6S,9R)-6-Isopropyl-3,9-dimethyl-1,4-dioxaspiro[4.5]decan-2-on, (1R,2S,5R)-N-(tert-Butyl)-2-isopropyl-5-methyl-cyclohexancarboxamid und Gemischen, umfassend zwei oder mehrere von diesen, ausgewählt ist.

7. Verwendung einer Zusammensetzung, wie definiert in einem der vorhergehenden Ansprüche, um eine wärmende Empfindung für den Mund, den Rachen und die Brust eines Konsumenten zu liefern.

8. Nahrungsmittelprodukt, Mundpflegeprodukt, Körperpflegeprodukt oder pharmazeutisches Produkt, umfassend die wärmend wahrgenommene Zusammensetzung gemäß einem der Ansprüche 1 bis 6.

9. Produkt gemäß Anspruch 8, in der Form eines flüssigen pharmazeutischen Produkts, eines Getränks, eines alkoholischen Getränks, eines Milchprodukts, eines Sojaprodukts, einer Suppe, eines Dressings, einer Soße oder eines Dips.

10. Produkt gemäß Anspruch 9 in der Form einer flüssigen analgetischen Zubereitung.

## Revendications

1. Composition à sensation de réchauffement comprenant
(i) un composant chaud,
(ii) un composant rafraîchissant,
(iii) éventuellement, un composant amer, et
(iv) un solvant pour les composants (i) à (iii)
dans laquelle le rapport pondéral de (i) à (ii) est de 1:3 à 1:35 et, si le composant rafraîchissant comprend du menthol ou un composé à arôme mentholé, ledit menthol ou composé à arôme mentholé est présent en une proportion de 2% en poids ou moins de la composition à sensation de réchauffement, à la condition que le composant amer (iii) soit présent à raison de moins de 0,15% en poids rapporté au poids total de la composition, et le solvant (iv) comporte moins de 10% en poids d'éthanol et de 20 à 99,9% de propylèneglycol, rapporté au poids total de la composition à sensation de réchauffement.

2. Composition selon la revendication 1, dans laquelle le rapport pondéral de (i) à (ii) est de 1:7 à 1:35.

3. Composition selon la revendication 1 ou la revendication 2, dans laquelle la quantité totale de (i) et de (ii) est d'au moins 0,3% en poids, rapporté au poids total d'un produit consommable dans lequel elle est présente.

4. Composition selon la revendication 1 ou la revendication 2, dans laquelle la quantité totale de (i) et de (ii) n'est pas supérieure à 1% en poids, rapporté au poids total d'un produit consommable dans lequel elle est présente.

5. Composition selon l'une quelconque des revendications précédentes, dans laquelle le composant chaud est sélectionné dans le groupe constitué par la pipérine, le pélargonyl-vanillylamide, le vanillylbutylamide, l'éther butylique de vanilline, l'eugénol, le gingérol, le polygodial, le shogaol, l'acétate de galangal, la capsaïcine (N-(4-hydroxy-3-méthoxybenzyl)-8-méthylnonanamide et/ou (6E)-N-(4-hydroxy-3-méthoxybenzyl)-8-méthyl-6-nonénamide), les analogues de la capsaïcine et les mélanges comprenant deux ou plus d'entre eux.

6. Composition selon l'une quelconque des revendications précédentes, dans laquelle le composant rafraîchissant est sélectionné dans le groupe constitué par le menthol, le succinate de menthyle, le lactate de menthyle, le p-menthane-3,8-diol, le 8-p-menthén-3-ol, le 3-(3'-p-menthanyloxy)-1,2-propanediol, le menthone glycérol cétal, la 2-(1-méthylpropyl)-1-cyclohexanone, le N-éthyl-3-p-menthanecarboxamide, l'acide aspartique, le N-(4-hydroxy-3-méthoxybenzyl)nonanamide, la 5-[5-(1,3-benzodioxol-5-yl)-1-(1-pipéridinyl)-2,4-pentadién-1-one, la 6-isopropyl-3,9-diméthyl-1,4-dioxaspiro[4.5]décan-2-one, le 2-isopropyl-N,2,3-triméthylbutanamide, et, le 7-isopropyl-4,10-diméthyl-tricyclo[4.4.0.0(1,5)]décan-4-ol, le 3-menthyl-3,6-dioxaheptanoate, le méthoxyacétate de 3-menthyle, le 3-menthyl-3,6,9-trioxadécanoate, le 3-menthyl-(2-hydroxyéthoxy)acétate, le menthyl-11-hydroxy-3,6,9-trioxadécanoate, la (3S,5R,6S,9R)-6-isopropyl-3,9-diméthyl-1,4-dioxaspiro[4.5]décan-2-one, la (3S,5S,6S,9R)-6-isopropyl-3,9-diméthyl-1,4-dioxaspiro[4.5]décan-2-one, le (1R,2S,5R)-2-isopropyl-5-méthylcyclohexanecarboxylate de 2,3-dihydroxypropyle, la (3S,5R,6S,9R)-6-isopropyl-3,9-diméthyl-1,4-dioxaspiro[4.5]décan-2-one, la (3S,SS,6S,9R)-6-isopropyl-3,9-diméthyl-1,4-dioxaspiro[4.5]décan-2-one, le (1R,2S,5R)-N-(tert-butyl)-2-isopropyl-5-méthylcyclohexanecarboxamide et les mélanges comprenant deux ou plus d'entre eux.

7. Utilisation d'une composition telle que définie dans l'une quelconque des revendications précédentes pour apporter une sensation de réchauffement dans la bouche, la gorge, et la poitrine d'un consommateur.

8. Produit alimentaire, produit de soin buccal, produit de soin corporel ou produit pharmaceutique comprenant la composition à sensation de réchauffement selon n'importe lesquelles des revendications 1 à 6.

9. Produit selon la revendication 8, sous forme de produit pharmaceutique liquide, boisson, boisson alcoolisée, produit laitier, produit à base de soja, soupe, assaisonnement, sauce ou trempette.

10. Produit selon la revendication 9 sous forme de préparation analgésique liquide.
